# EUROPEAN PATENT APPLICATION

(11) **EP 3 711 761 A1**
(43) Date of publication of application: **23.09.2020**
(21) Application number: 18879735.1
(22) Date of filing: 13.11.2018
(51) Int. Cl.: A61K 31/41, A61K 31/16

(54) **USE OF CARBAMATE COMPOUND FOR REDUCING OR TREATING DEVELOPMENTAL DISORDERS INCLUDING FRAGILE X SYNDROME, ANGELMAN SYNDROME OR RETT SYNDROME**

(30) Priority: 14.11.2017 KR 20170151251
(71) Applicant: SK Biopharmaceuticals Co., Ltd., Gyeonggi-do 13494 (KR)
(72) Inventor: SHIN, Hye Won, Seongnam-si Gyeonggi-do 13494 (KR); HWANG, Sun Gwan, Seongnam-si Gyeonggi-do 13494 (KR)
(74) Representative: Vossius & Partner Patentanwälte Rechtsanwälte mbB
(86) International application number: PCT/KR2018/013765
(87) International publication number: WO 2019/098630

(57) **Abstract**

The present invention relates to a use of a carbamate compound represented by chemical formula 1, or a pharmaceutically acceptable salt, a solvate or a hydrate thereof for reducing or treating developmental disorders including fragile X syndrome, Angelman syndrome or Rett syndrome.

## Description

### [Technical Field]

The present invention relates to use of a carbamate compound of the following Formula 1, or a pharmaceutically acceptable salt, solvate or hydrate thereof for alleviating or treating developmental disorder including fragile X syndrome, Angelman syndrome or Rett syndrome: 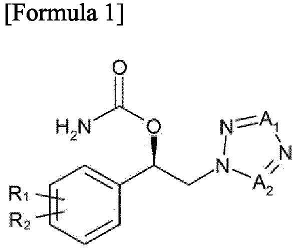 wherein,
R₁, R₂, A₁ and A₂ are as defined herein.

### [Background Art]

Treatment for developmental disorders such as fragile X syndrome (FXS), Rett syndrome and Angelman syndrome is limited.

Fragile X syndrome (FXS) is a genetic disorder that is the most common single gene cause of autism, especially the genetic cause of intellectual disability among boys. Fragile X syndrome is caused by mutations in the fragment X mental retardation 1 (Fmr1) gene located on the X chromosome. The Fmr1 gene is the first identified autism-related gene and encodes a fragment X mental retardation protein (FMRP), an RNA binding protein that regulates translation. That is, a protein FMRP is made by the Fmr1 gene-which is necessary for normal brain development, and this protein is not sufficiently made in fragile X syndrome (Source: Center for Disease Control and Prevention). This functional loss typically occurs when there is an expansion of CGG trinucleotide repeats in the 5' untranslated region of the Fmr1 gene. This extension appears as a weak or "fragile-like" end on the X chromosome.

Fragile X syndrome occurs in both men and women, but the symptoms of women are relatively mild compared to those of men, and the incidence is higher in men than in women. According to another report, this disorder occurs in 1 in 4,600 men and 1 in 8,000 women (Source: Genetics Home Reference, National Library of Medicine).

Symptoms of fragile X syndrome include developmental delays, learning disability, and sociobehavioral disorders (mismatched eyes, anxiety, attention problems, flapping hands, talking or acting without thinking, excessively active). Men have moderate to severe intellectual disability, and some women have normal category intelligence or some have intellectual disability. Autism spectrum disorder occurs frequently in people with fragile X syndrome (Source: Center for Disease Control and Prevention). In addition, there is a risk of seizure in fragile X syndrome, and it has been known that about 14% of men and about 4% of women experience seizures (Berry-Kravis et al., 2010, "Seizures in Fragile X Syndrome: Characteristics and Comorbid Diagnoses," Am J Intellect Dev Disabil. 115 (6): 461-72).

In order to diagnose fragile X syndrome, an abnormality of the FMR1 gene is diagnosed through a blood DNA test.

Up to now, there is no fundamental treatment for FXS. Adequate education can help, and sometimes medications are used for alleviating behavioral disorders and seizures.

Angelman syndrome is a disease that occurs when a specific gene on chromosome 15 is not genetically inherited. Symptoms of Angelman syndrome include developmental delays, intellectual disability, sociobehavioral disorders (easily excited personality, excessively active, attention problems) and seizures.

In addition, autism categorical disorders frequently occur in people with Angelman syndrome. Up to now, there is no fundamental treatment for Angelman syndrome. Appropriate education can help, and sometimes medications are used for alleviating the symptoms of Angelman syndrome described above.

Rett syndrome is a disease caused by genetic mutation of the MECP2 gene. Symptoms of Rett Syndrome include developmental delays, intellectual disability, sociobehavioral disorders (easily excited personality, excessively active, attention problems) and seizures.

In addition, people with Rett syndrome exhibit symptoms similar to autism categorical disorders. Up to now, there is no fundamental treatment for Rett syndrome. Adequate education can help, and sometimes medications are used for alleviating the symptoms of Rett syndrome described above.

That is, fragile X syndrome, Angelman syndrome and Rett syndrome have common symptoms of developmental delays, intellectual disability, sociobehavioral disorders, seizure and autism categorical disorder (or similar symptoms). In addition, fragile X syndrome, Angelman syndrome and Rett syndrome have no fundamental treatment, and thus have a common feature in that drugs are administered for the purpose of alleviating their symptoms.

### [Disclosure of the Invention]

### [Problem to be Solved]

The present invention is intended to provide a method for the alleviation or treatment of developmental disorder.

The present invention is also intended to provide the use of a therapeutically effective amount of a carbamate compound of the following Formula 1, or a pharmaceutically acceptable salt, solvate or hydrate thereof, for the alleviation or treatment of developmental disorder: 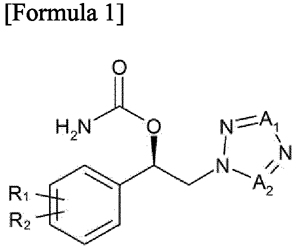 wherein,
R₁, R₂, A₁ and A₂ are as defined herein.

The present invention is intended to provide a method for the alleviation or treatment of fragile X syndrome.

The present invention is also intended to provide the use of a carbamate compound of the above Formula 1, or a pharmaceutically acceptable salt, solvate or hydrate thereof, for the alleviation or treatment of fragile X syndrome.

The present invention is also intended to provide a method for the alleviation or treatment of Angelman syndrome.

The present invention is also intended to provide the use of a carbamate compound of the above Formula 1, or a pharmaceutically acceptable salt, solvate or hydrate thereof, for the alleviation or treatment of Angelman syndrome.

The present invention is also intended to provide a method for the alleviation or treatment of Rett syndrome.

The present invention is also intended to provide the use of a carbamate compound of the above Formula 1, or a pharmaceutically acceptable salt, solvate or hydrate thereof, for the alleviation or treatment of Rett syndrome.

### [Technical Solution to the Problem]

The present invention provides a medicament for the alleviation or treatment of developmental disorder, comprising a therapeutically effective amount of a carbamate compound of the following Formula 1, or a pharmaceutically acceptable salt, solvate or hydrate thereof: 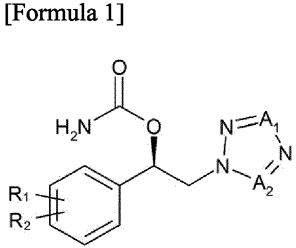 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

In addition, the present invention provides a method for alleviating or treating developmental disorder, in a subject, comprising administering to the subject a therapeutically effective amount of the carbamate compound of the above Formula 1, or a pharmaceutically acceptable salt, solvate or hydrate thereof.

In addition, the present invention provides the use of the carbamate compound of the above Formula 1, or a pharmaceutically acceptable salt, solvate or hydrate thereof for the alleviation or treatment of developmental disorder.

In addition, the present invention provides a medicament for the alleviation or treatment of fragile X syndrome, comprising a therapeutically effective amount of the carbamate compound of the above Formula 1, or a pharmaceutically acceptable salt, solvate or hydrate thereof

In addition, the present invention provides a method for alleviating or treating fragile X syndrome, in a subject, comprising administering to the subject a therapeutically effective amount of the carbamate compound of the above Formula 1, or a pharmaceutically acceptable salt, solvate or hydrate thereof.

In addition, the present invention provides the use of the carbamate compound of the above Formula 1, or a pharmaceutically acceptable salt, solvate or hydrate thereof for the alleviation or treatment of fragile X syndrome.

In addition, the present invention provides a medicament for the alleviation or treatment of Angelman syndrome, comprising a therapeutically effective amount of the carbamate compound of the above Formula 1, or a pharmaceutically acceptable salt, solvate or hydrate thereof

In addition, the present invention provides a method for alleviating or treating Angelman syndrome, in a subject, comprising administering to the subject a therapeutically effective amount of the carbamate compound of the above Formula 1, or a pharmaceutically acceptable salt, solvate or hydrate thereof.

In addition, the present invention provides the use of the carbamate compound of the above Formula 1, or a pharmaceutically acceptable salt, solvate or hydrate thereof for the alleviation or treatment of Angelman syndrome.

In addition, the present invention provides a medicament for the alleviation or treatment of Rett syndrome, comprising a therapeutically effective amount of the carbamate compound of the above Formula 1, or a pharmaceutically acceptable salt, solvate or hydrate thereof

In addition, the present invention provides a method for alleviating or treating Rett syndrome, in a subject, comprising administering to the subject a therapeutically effective amount of the carbamate compound of the above Formula 1, or a pharmaceutically acceptable salt, solvate or hydrate thereof.

In addition, the present invention provides the use of the carbamate compound of the above Formula 1, or a pharmaceutically acceptable salt, solvate or hydrate thereof for the alleviation or treatment of Rett syndrome.

According to one embodiment of the present invention, in the above Formula 1, R₁ and R₂ are each independently selected from the group consisting of hydrogen, halogen and C₁-C₈ alkyl.

In one embodiment of the present invention, the halo-C₁-C₈ alkyl is perfluoroalkyl.

According to another embodiment of the present invention, the carbamate compound of the above Formula 1 is carbamic acid (R)-1-(2-chlorophenyl)-2-tetrazol-2-yl-ethyl ester of the following Formula 2:

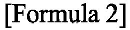

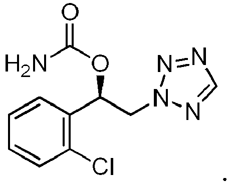

A person having ordinary skill in the art of synthesis of compounds could have easily prepared the carbamate compounds of the above Formulas 1 and 2 using known compounds or compounds which can be easily prepared therefrom. Specifically, methods for preparing the compounds of the above Formula 1 are described in detail in International Publication Nos. WO 2006/112685 A1, WO 2010/150946 A1 and WO 2011/046380 A2, the disclosures of which are incorporated herein by reference. The compounds of the above Formula 1 can be chemically synthesized by any of the methods described in the above documents, but the methods are merely exemplary ones, and the order of the unit operation and the like may be selectively changed if necessary. Hence, the above methods are not intended to limit the scope of the invention.

The carbamate compounds of the above Formula 1 can be used for the alleviation or treatment of developmental disorder including fragile X syndrome, Angelman syndrome or Rett syndrome.

The carbamate compounds of the above Formula 1 can be used for the prevention, alleviation or treatment of symptoms of developmental disorder including fragile X syndrome, Angelman syndrome or Rett syndrome.

Symptoms of developmental disorder include developmental delays, learning disability, sociobehavioral disorders (mismatched eyes, anxiety, attention problems, flapping hands, talking or acting without thinking, excessively active) or seizures.

Symptoms of fragile X syndrome include developmental delays, learning disability, and sociobehavioral disorders (mismatched eyes, anxiety, attention problems, flapping hands, talking or acting without thinking, excessively active). Men have moderate to severe intellectual disabilities, and some women have normal category intelligence or some have intellectual disabilities. Autism spectrum disorder occurs frequently in people with fragile X syndrome (Source: Center for Disease Control and Prevention). In addition, there is a risk of seizure in fragile X syndrome, and it has been known that about 14% of men and about 4% of women experience seizures (Berry-Kravis et al., 2010, "Seizures in Fragile X Syndrome: Characteristics and Comorbid Diagnoses," Am J Intellect Dev Disabil. 115 (6): 461-72).

Symptoms of Angelman syndrome and Rett syndrome include developmental delays, intellectual disability, sociobehavioral disorders (easily excited personality, excessively active, attention problems) and seizures.

Therefore, the medicament and pharmaceutical composition according to the present invention can be used for the prevention, alleviation or treatment of symptoms of developmental disorder including fragile X syndrome, Angelman syndrome or Rett syndrome, and the symptoms include, but are not limited to, developmental delays, learning disability, sociobehavioral disorders and seizures.

In addition, the medicament according to the present invention can be used for the alleviation or treatment of autism spectrum disorders caused by fragile X syndrome, Angelman Syndrome and Rett Syndrome, or autism spectrum disorders showing symptoms similar to fragile X syndrome, Angelman Syndrome and Rett Syndrome.

The efficacy of the compound of the above Formula 1 on developmental disorders including fragile X syndrome, Angelman syndrome or Rett syndrome can be confirmed by the use of known models. For example, Fmrl gene-deficient mouse model represents several clinical symptoms observed in developmental disorders such as Fragile X Syndrome, Angelman Syndrome or Rett Syndrome, and has been used as a means to verify drug efficacy for the study of disease mechanisms and development of therapeutic agents (Bakker et al., 1994, "Fmr1 knockout mice: A model to study fragile X mental retardation", Cell, 15; 78 (1): 23-33). Typical phenotypes of this mouse model include audiogenic seizure, excessive locomotor activity, cognitive deficit, attention problem and the like.

The dosage of the carbamate compounds of Formula 1 for the alleviation or treatment of the above diseases may typically vary depending on the severity of the disease, the body weight and the metabolic status of the subject. A "therapeutically effective amount" for an individual patient refers to an amount of the active compound sufficient to achieve the above pharmacological effect, i.e., the therapeutic effect as described above. The therapeutically effective amount of the compound of Formula 1 is 50 to 500 mg, 50 to 400 mg, 50 to 300 mg, 100 to 400 mg, 100 to 300 mg, 50 to 200 mg, or 100 to 200 mg, based on the free form and once-daily administration to humans.

The compounds of the present invention may be administered by any conventional method used for administration of a therapeutic agent, such as oral, parenteral, intravenous, intramuscular, subcutaneous or rectal administration.

The medicament or pharmaceutical composition according to one embodiment of the present invention may comprise a therapeutically effective amount of a compound selected from the group consisting of the carbamate compounds of the present invention, their pharmaceutically acceptable salts, solvates, hydrates and combinations thereof.

Examples of the pharmaceutically acceptable salts of the carbamate compounds of the above Formula 1 include independently, acetate, benzenesulfonate, benzoate, bitartrate, calcium acetate, camsylate, carbonate, citrate, edetate, edisylate, estolate, esylate, fumarate, gluceptate, gluconate, glutamate, glycoloyl arsanilate, hexylresorcinate, hydravamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate, oxalate or hemi-tartrate, teoclate, triethiodide, benzathine, chloroprocaine, choline, diethanolamine, ethylenediamine, meglumine, procaine, aluminum, ammonium, tetramethylammonium, calcium, lithium, magnesium, potassium, sodium and zinc.

The medicament or pharmaceutical composition according to one embodiment of the present invention may be administered orally or parenterally. The parenteral administration may include intravenous injection, subcutaneous injection, intramuscular injection, intraperitoneal injection, endothelial administration, topical administration, intranasal administration, intravaginal administration, intrapulmonary administration, rectal administration and the like. In the case of oral administration, the pharmaceutical composition according to one embodiment of the present invention may be formulated as a plain tablet (uncoated tablet) or such that the active agent is coated or it is protected against degradation in the stomach. In addition, the composition can be administered by any device capable of transferring the active substance to a target cell. The route of administration may vary depending upon the general condition and age of the subject to be treated, the nature of the treatment condition and the active ingredient selected.

A suitable dosage of the medicament or pharmaceutical composition according to one embodiment of the present invention may vary depending on factors such as the formulation method, administration method, age, body weight and gender of patients, pathological condition, diet, administration time, administration route, excretion rate and reaction sensitivity, and doctors having ordinary skill can easily determine and prescribe dosages that are effective for the desired treatment or prophylaxis. The pharmaceutical composition according to one embodiment may be administered in one or more doses, for example, one to four times per day. The pharmaceutical composition according to one embodiment may contain the compounds of Formula 1 in the amount of 50 to 500 mg, 50 to 400 mg, 50 to 300 mg, 100 to 400 mg, 100 to 300 mg, 50 to 200 mg, or 100 to 200 mg, preferably 50 to 300 mg, more preferably 50 to 200 mg, based on the free form.

The medicament or pharmaceutical composition according to one embodiment of the present invention may be formulated using a pharmaceutically acceptable carrier and/or excipient according to a method that a person having ordinary skill in the art could easily carry out, thereby to be prepared in a unit dose form or to be contained in a multi-dose container. The above formulation may be a solution in oil or an aqueous medium, a suspension or an emulsion (emulsified solution), an extract, a powder, granules, a tablet, or a capsule, and may further include a dispersing or stabilizing agent. In addition, the pharmaceutical composition may be administered in the form of suppositories, sprays, ointments, creams, gels, inhalants or skin patches. The pharmaceutical composition may also be prepared for mammalian administration, more preferably for human administration.

Pharmaceutically acceptable carriers may be solid or liquid, and may be one or more selected from fillers, antioxidants, buffers, bacteriostats, dispersants, adsorbents, surfactants, binders, preservatives, disintegrants, sweeteners, flavors, glidants, release-controlling agents, wetting agents, stabilizers, suspending agents and lubricants. In addition, the pharmaceutically acceptable carriers may be selected from saline, sterile water, Ringer's solution, buffered saline, dextrose solution, maltodextrin solution, glycerol, ethanol and mixtures thereof.

In one embodiment, suitable fillers include, but are not limited to, sugar (e.g., dextrose, sucrose, maltose and lactose), starch (e.g., corn starch), sugar alcohol (e.g., mannitol, sorbitol, maltitol, erythritol and xylitol), starch hydrolysate (e.g., dextrin and maltodextrin), cellulose or cellulose derivatives (e.g., microcrystalline cellulose) or mixtures thereof.

In one embodiment, suitable binders include, but are not limited to, povidone, copovidone, methylcellulose, hydroxypropylmethylcellulose, hydroxypropylcellulose, hydroxyethylcellulose, gelatin, gum, sucrose, starch or mixtures thereof.

In one embodiment, suitable preservatives include, but are not limited to, benzoic acid, sodium benzoate, benzyl alcohol, butylated hydroxyanisole, butylated hydroxytoluene, chlorbutol, gallate, hydroxybenzoate, EDTA or mixtures thereof.

In one embodiment, suitable disintegrants include, but are not limited to, sodium starch glycolate, cross-linked polyvinylpyrrolidone, cross-linked carboxymethylcellulose, starch, microcrystalline cellulose or mixtures thereof.

In one embodiment, suitable sweeteners include, but are not limited to, sucralose, saccharin, sodium saccharin, potassium saccharin, calcium saccharin, acesulfame potassium or sodium cyclamate, mannitol, fructose, sucrose, maltose or mixtures thereof.

In one embodiment, suitable glidants include, but are not limited to, silica, colloidal silicon dioxide, talc and the like.

In one embodiment, suitable lubricants include, but are not limited to, long chain fatty acids and salts thereof, such as magnesium stearate and stearic acid, talc, glyceride wax or mixtures thereof.

As used herein, the terms "prevent," "preventing" and "prevention" refer to reducing or eliminating the likelihood of a disease.

As used herein, the terms "alleviate," "alleviating" and "alleviation" refer to ameliorating a disease and/or its accompanying symptoms altogether or in part.

As used herein, the terms "treat," "treating" and "treatment" refer to eliminating a disease and/or its accompanying symptoms altogether or in part.

As used herein, the term "subject" refers to an animal that is the object of therapy, observation or experiment, preferably a mammal (such as primates (e.g., a human), cattle, sheep, goats, horses, dogs, cats, rabbits, rats, mice, etc.), most preferably a human.

As used herein, the term "therapeutically effective amount" refers to the amount of active compound or pharmaceutical formulation that elicits a biological or medical response in the system, animal or human, including alleviation of the symptoms of the disease or disorder to be treated, wherein said amount is sought by a researcher, veterinarian, doctor (physician) or other clinician.

As used herein, the term "composition" encompasses a product that contains a specified amount of a particular ingredient and any product that results directly or indirectly from a combination of specified amounts of particular ingredients.

### [Effect of the Invention]

The medicament and the pharmaceutical composition according to the present invention can effectively alleviate and treat developmental disorder such as fragile X syndrome, Angelman syndrome or Rett syndrome.

### [Brief Description of the Drawings]

Figure 1 shows the results of the suppressive effects of Test Compound (10 mg/kg, 20 mg/kg) and the positive control group (MPEP, 2-methyl-6-(phenylethynyl)pyridine) on the occurrence of seizures in an audiogenic seizure test performed by the use of an Fmrl gene-deficient mouse model.
Figure 2 shows the results of the preventive effects of Test Compound (10 mg/kg, 20 mg/kg) and the positive control group (MPEP) on respiratory arrest due to seizure in an audiogenic seizure test performed by the use of an Fmr1 gene-deficient mouse model.
Figure 3 shows the results of the regulatory effects of Test Compound (10 mg/kg, 20 mg/kg) and the positive control group (MPEP) on seizure score in an audiogenic seizure test performed by the use of an Fmr1 gene-deficient mouse model.
Figure 4 shows the results of the effects of Test Compound (10 mg/kg, 20 mg/kg) and the positive control group (MPEP) on mouse survival in an audiogenic seizure test performed by the use of an Fmr1 gene-deficient mouse model.
Figure 5 shows the results of the effects of Test Compound (10 mg/kg, 20 mg/kg) and the positive control group (MPEP) on the percent seized in an audiogenic seizure test performed by the use of an Fmr1 gene-deficient mouse model.
Figure 6 shows the results of the effects of Test Compound (10 mg/kg, 20 mg/kg) on the locomotor activity in an Fmr1 gene-deficient mouse model.
Figure 7 shows the results of the effects of Test Compound (10 mg/kg, 20 mg/kg) on the increased rearing activity in an Fmr1 gene-deficient mouse model.
Figure 8 shows the results of the effects of Test Compound (10 mg/kg, 20 mg/kg) on the reduced fear conditioning in a contextual fear conditioning test by the use of an Fmrl gene-deficient mouse model.
Figure 9 shows the results of the effects of Test Compound (10 mg/kg, 20 mg/kg) on the reduced fear conditioning in a cued fear conditioning test by stimulus signal using an Fmr1 gene-deficient mouse model.

### [Specific Embodiments to Carry Out the Invention]

Hereinafter, the present invention will be explained in more detail through working examples. However, the following working examples are only intended to illustrate one or more embodiments and are not intended to limit the scope of the invention.

### Preparation Example: Synthesis of carbamic acid (R)-1-(2-chlorophenyl)-2-tetrazol-2-yl-ethyl ester

Carbamic acid (R)-1-(2-chlorophenyl)-2-tetrazol-2-yl-ethyl ester (Test Compound) was prepared according to the method described in Preparation Example 50 of International Publication No. WO 2010/150946.

### Example 1: Anticonvulsant efficacy test for audiogenic seizure in Fmrl gene-deficient mouse model

The Fmr1 gene knockout mouse model lacks the FMRP protein due to the destruction of the Fmrl gene, and the typical phenotype of this mouse is audiogenic seizure, excessive locomotor activity, cognitive deficit, attention problem and the like. As such, the Fmr1 gene-deficient mouse model shows several clinical symptoms observed in developmental disorders such as fragile X syndrome, Angelman syndrome or Rett syndrome, and has been used as a means to verify drug efficacy for researching developmental disorders and development of therapeutic agents (Kazdoba et al., 2014, "Modeling fragile X syndrome in the Fmrl knockout mouse", Intractable Rare Dis Res. 2014 Nov; 3(4): 118-133; Bakker et al., 1994, "Fmrl knockout mice: A model to study fragile X mental retardation", Cell, 15; 78(1): 23-33).

### Experimental animals

Male FVB.129P2-*Pde6b*⁺ *Tyr^{c-ch} Fmr1^{tm1Cgr}*/J gene-deficient mice were purchased from Jackson Laboratories, and audiogenic seizure experiments were performed at 3 weeks of age. After purchase, the experimental animals were maintained under a controlled lighting environment (12 hours lighting/12 hours non-lighting) and maintained at a temperature of 20-23°C and about 50% relative humidity. Food and water were provided *ad libitum.* Mice were randomly divided into groups. The test was carried out under lighting.

### Drug

MPEP (2-methyl-6-(phenylethynyl)pyridine) HCl was used as a positive control, dissolved in sterile injectable saline, and administered intraperitoneally at a dose of 30 mg/kg. MPEP was prepared at a volume of 10 ml/kg and administered 30 minutes before the test.

Test Compound was dissolved in 30% PEG 300, and two doses of 10 mg/kg and 20 mg/kg were used. Test Compound was prepared at a volume of 10 ml/kg and administered intraperitoneally 30 minutes before the test. As a negative control, a vehicle (30% PEG 300) was administered.

### Treatment Groups

1. Fmr1 gene-deficient mouse - vehicle administration
2. Fmr1 gene-deficient mouse - MPEP 30 mg/kg administration
3. Fmr1 gene-deficient mouse - Test Compound 10 mg/kg administration
4. Fmr1 gene-deficient mouse - Test Compound 20 mg/kg administration

Ten mice per group were used.

### Audiogenic seizure test

Vehicle, MPEP or Test Compound was administered to mice of 21 days of age 30 minutes before the test. Mice were placed in a Plexiglas chamber for 15 seconds and exposed to 135 decibels (dB) of sound. During the test for 5 minutes, the mice were scored as following criteria based on the reaction of the mouse, the time required until the reaction and the intensity of the seizure.
0: No response
1: Wild running and jumping
2: Clonic seizures
3: Clonic-tonic seizures
4: Tonic seizures
5: Respiratory arrest

The following endpoints were measured and observed, and in the case of animals that had no reaction, the time to reaction was set to 300 seconds for convenience in analysis.
1. Time to seizure occurrence (300 seconds if seizure does not occur)
2. Time to respiratory arrest (300 seconds if mouse survives without respiratory arrest)
3. Seizure score
4. Percent survival
5. Percent seized

### Statistical analysis

Data were analyzed by ANOVA, and if appropriate, Fisher PLSD post-hoc analysis was performed. The average seizure intensity was analyzed non-parametrically. The effect was defined as significant when p <0.05. Results are represented as mean values ± standard error.

### Test results

### 1. Weight

When the body weight measured before drug administration on the test day was analyzed by 1-way ANOVA, there was no significant difference by group.

### 2. Time to seizure occurrence

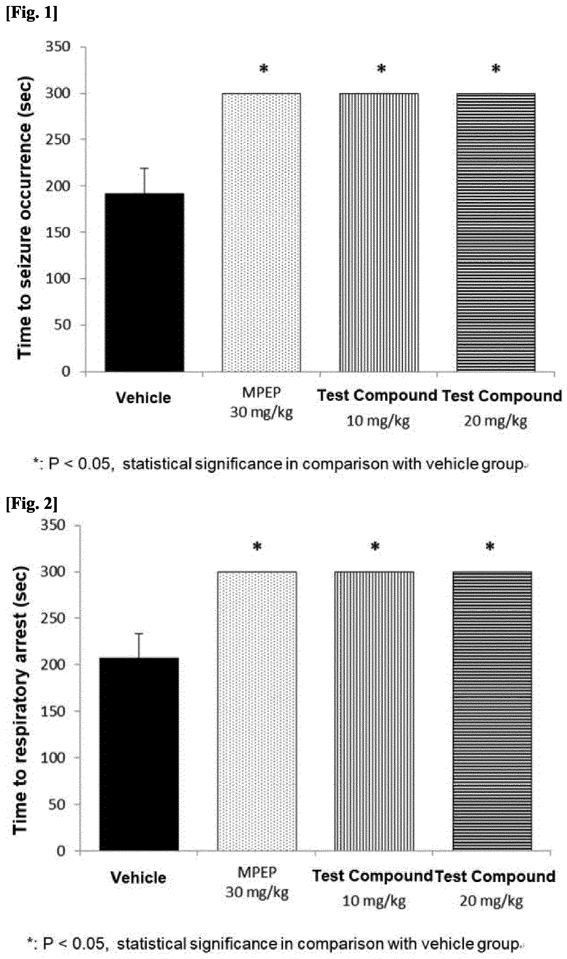

The groups in which MPEP (30 mg/kg) and Test Compound (10 mg/kg, 20 mg/kg) were administered significantly increased the time required to develop seizures compared to the vehicle administration group. Mice in the MPEP and Test Compound administration groups did not cause seizures, and thus the time required for seizure occurrence was recorded as 300 seconds (Fig. 1).

### 3. Time to respiratory arrest

The groups in which MPEP (30 mg/kg) and Test Compound (10 mg/kg, 20 mg/kg) were administered significantly increased the time required to respiratory arrest compared to the vehicle administration group. Mice in the MPEP and Test Compound administration groups did not cause respiratory arrest, and thus the time required to respiratory arrest was recorded as 300 seconds (Fig. 2).

### 4. Median seizure score

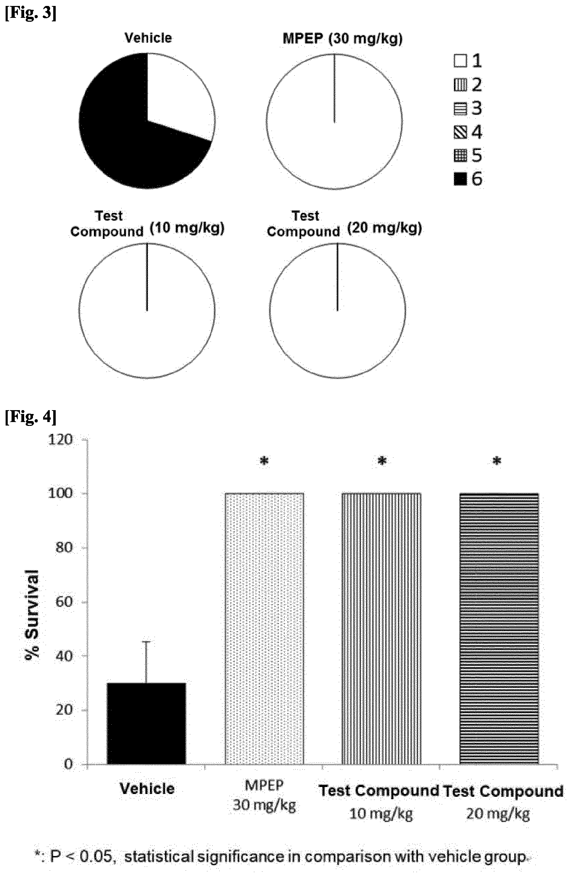

In the groups in which MPEP (30 mg/kg) and Test Compound (10 mg/kg, 20 mg/kg) were administered, the seizure intensity level of the mice was significantly reduced compared to the vehicle administration group (Fig. 3).

### 5. Percent survival

In the groups in which MPEP (30 mg/kg) and Test Compound (10 mg/kg, 20 mg/kg) were administered, the viability of mice was significantly increased compared to the vehicle administration group, and 100% of mice in the MPEP and Test Compound administration groups survived (Fig. 4).

### 6. Percent seized

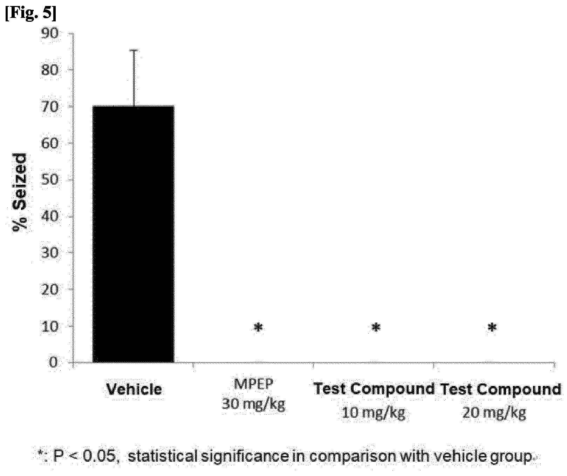

No seizures occurred in the groups in which MPEP (30 mg/kg) and Test Compound (10 mg/kg, 20 mg/kg) were administered (Fig. 5).

### Example 2: Open field test and fear conditioning test in Fmrl gene-deficient mouse model

### Experimental animals

Male FVB.129P2-*Pde6b*⁺ *Tyr^{c-ch} Fmr1^{tm1Cgr}*/J gene-deficient mice were purchased from Jackson Laboratories. After purchase, the experimental animals were maintained under a controlled lighting environment (12 hours lighting/12 hours non-lighting) and maintained at a temperature of 20-23°C and about 50% relative humidity. Food and water were provided *ad libitum.* Mice were randomly divided into groups. The drugs were administered once a day for 6 days at 8 weeks of age. The test was carried out under lighting.

### Drug

Test Compound was dissolved in 30% PEG 300, and two doses of 10 mg/kg and 20 mg/kg were used. Test Compound was prepared at a volume of 10 ml/kg and administered intraperitoneally 30 minutes before the test.

### Test Group

1. Wild-type mouse - vehicle administration
2. Fmr1 gene-deficient mouse - vehicle administration
3. Fmr1 gene-deficient mouse - Test Compound 10 mg/kg administration
4. Fmr1 gene-deficient mouse - Test Compound 20 mg/kg administration

Ten mice per group were used.

### Open field test and fear conditioning test

1. Locomotor activity: Locomotor activity test was performed on day 4 of administration in the course of administering daily for 6 days in 8-week-old mice.
   A Plexiglas square chamber (27.3 × 27.3 × 20.3 cm) surrounded by infrared rays was used to measure the horizontal and vertical movements. Mice were acclimatized to the laboratory for at least 1 hour before the test and then placed in the center of the chamber to measure activity in the open field for 1 hour. The test performed space was cleaned with each mouse change. The distance was measured by the breakage of the horizontal light beam by the movement of the mouse. The hourly change of activity and rearing activity (hind legs standing) was expressed in 5-minute units. The total distance traveled and the total number of rearing activity were the sum of the values for 60 minutes.
2. Fear conditioning test: On day 5 and day 6 of administration, and 30 minutes after administration, training and drug efficacy tests were carried out, respectively. The test was performed using a fear conditioning system manufactured by Coulbourn Instruments (PA, USA).

Training was done 30 minutes after administration on day 5 of administration. The mice were placed in a conditioning chamber and accustomed to the environment for 120 seconds, and then exposed to 6 kHz, 75 dB sound stimulus (conditioned stimulus) for 20 seconds. 30 seconds after the end of the conditioned stimulation, 0.5 mA electrostimulation shock (unconditioned stimulus) was applied to the mouse paws. Mice were left in the conditioning chamber for an additional 60 seconds and then returned to the original cage.

On the morning of day 6, 30 minutes after administration mice were placed in the same chamber as the previous day, and then trained for 5 minutes without shock or other obstructions in order to perform a drug efficacy test for contextual memory. On day 6, after 4 hours of the contextual conditioning test, a drug efficacy test was performed on cued memory by stimulus signals. The mice were exposed to a new environment for 2 minutes (before the stimulation signal), and conditions and stimulation were applied three times in total for 20 seconds. Freezing behavior was defined as the complete loss of movement and was captured using a video system and FreezeView software (Coulbourn Instruments PA, USA). The chamber was cleaned with 70% ethanol with each mouse change.

### Statistical analysis

The data were analyzed by ANOVA, and if appropriate, Fisher PLSD post-hoc analysis was performed using Statview software. The effect was defined as significant when p <0.05. Results are represented as mean values ± standard error.

### Open field test results

### 1. Locomotor activity

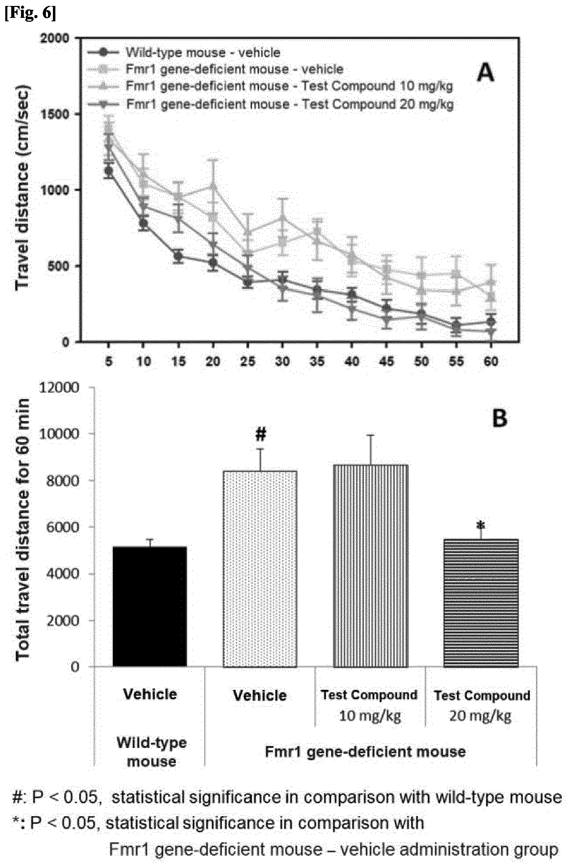

The change in activity over time for 60 minutes is shown in Figure 6A, and the sum of the total travel distances is shown in Figure 6B. As a result of ANOVA analysis, Fmrl gene-deficient mice in which the vehicle was administered showed significantly increased activity compared to wild-type mice. In addition, activity was significantly decreased in the Fmrl gene-deficient mice in which Test Compound (20 mg/kg) was administered in comparison with the vehicle administration group.

### 2. Rearing

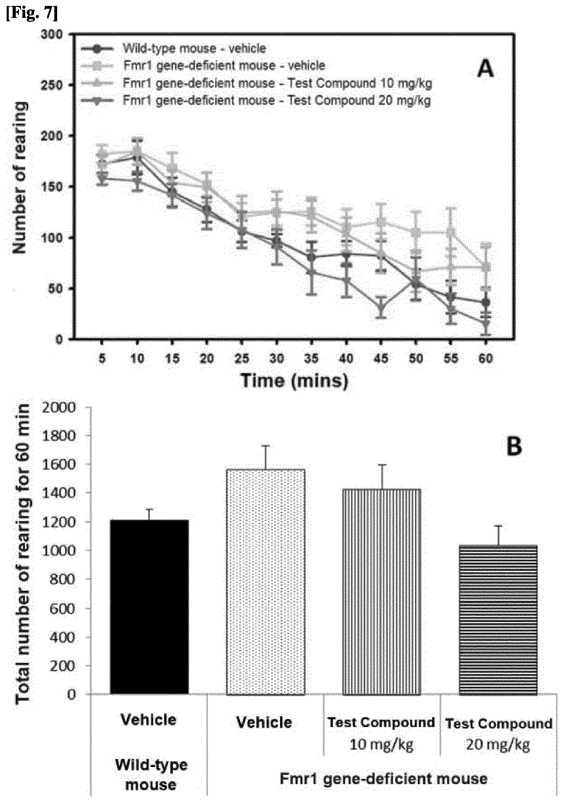

The hourly change in frequency of rearing activity for 60 minutes is shown in Figure 7A, and the total number of rearing activity is shown in Figure 7B. As a result of ANOVA analysis, Fmr1 gene-deficient mice in which the vehicle was administered showed increased number of rearing activity compared to wild-type mice, but it was not significant. In addition, rearing activity was decreased in the Fmr1 gene-deficient mice in which Test Compound (20 mg/kg) was administered in comparison with the vehicle administration group.

### Fear conditioning test results

### 1. Contextual fear conditioning

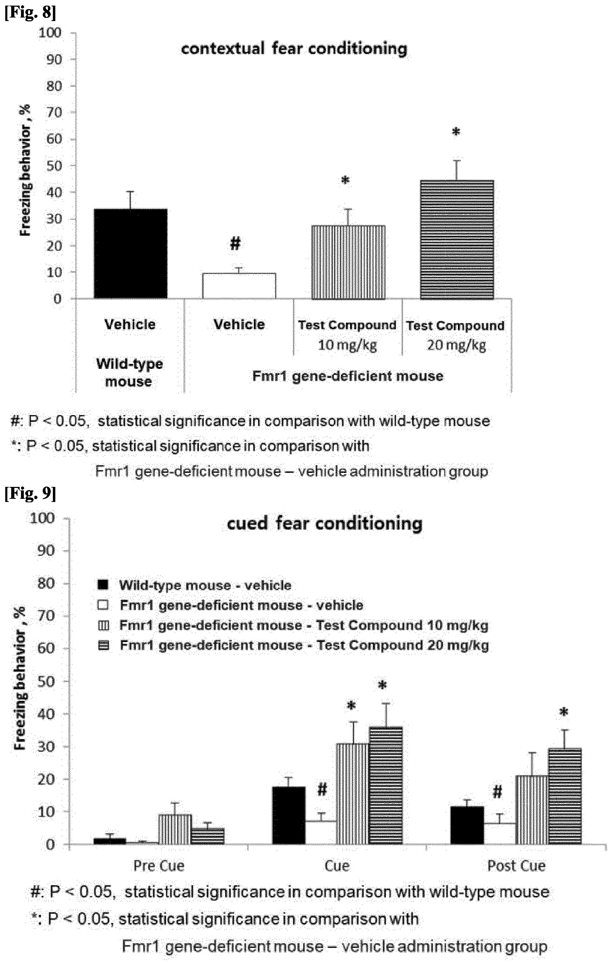

The percentage of freezing during the contextual fear conditioning test is represented in Figure 8. As a result of ANOVA analysis, Fmrl gene-deficient mice in which the vehicle was administered showed significantly reduced freezing compared to wild-type mice. In addition, freezing was significantly increased in the Fmr1 gene-deficient mice in which Test Compound (10 mg/kg, 20 mg/kg), which is similar to wild-type mice, in comparison with the vehicle administration group.

### 2. Fear conditioning test by stimulus signal (Cue test)

The percentage of freezing during the fear conditioning test by stimulus signal is represented in Figure 9. When all groups were analyzed, there were no significant differences between wild-type mice and Fmr1 gene-deficient mice. However, t-test showed significant differences between wild-type mice and Fmr1 gene-deficient mice. In the Fmr1 gene-deficient mice, a 1-way ANOVA analysis was performed to compare Test Compound administration group with the vehicle administration group. When the time of giving the stimulus signal was compared with the time after stimulus signal, there was a significant effect of the administration in the rate of freezing. While the stimulus signal was given, Test Compound significantly increased the rate of freezing in Fmr1 gene-deficient mice at both doses. After the stimulus signal, there was a significant increase in freezing at the dose of 20 mg/kg Test Compound.

## Claims

1. A medicament for the alleviation or treatment of developmental disorder, comprising a therapeutically effective amount of a carbamate compound of the following Formula 1, or a pharmaceutically acceptable salt, solvate or hydrate thereof: 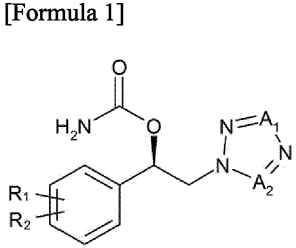 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

2. The medicament according to Claim 1, wherein the developmental disorder is fragile X syndrome, Angelman syndrome or Rett syndrome.

3. The medicament according to Claim 1, which is for use in the prevention, alleviation or treatment of symptoms of developmental disorder.

4. The medicament according to Claim 1, wherein the symptom of developmental disorder is developmental delay, learning disability, sociobehavioral disorder or seizure.

5. The medicament according to any one of Claims 1 to 4, wherein the therapeutically effective amount of the carbamate compound of Formula 1 is 50 to 500 mg based on the free form once-daily administration.

6. A medicament for the alleviation or treatment of fragile X syndrome, comprising a therapeutically effective amount of a carbamate compound of the following Formula 1, or a pharmaceutically acceptable salt, solvate or hydrate thereof: 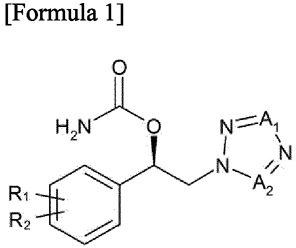 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

7. The medicament according to Claim 6, wherein the carbamate compound of Formula 1 is carbamic acid (R)-1-(2-chlorophenyl)-2-tetrazol-2-yl-ethyl ester of the following Formula 2:

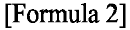

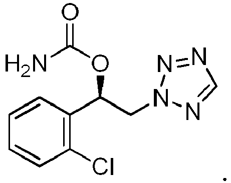

8. The medicament according to Claim 6, which is for use in the prevention, alleviation or treatment of symptoms of fragile X syndrome.

9. The medicament according to Claim 8, wherein the symptom of fragile X syndrome is developmental delay, learning disability, sociobehavioral disorder or seizure.

10. The medicament according to Claim 6, which is for use in the alleviation or treatment of autism spectrum disorder caused by fragile X syndrome, or autism spectrum disorder showing symptoms similar to fragile X syndrome.

11. The medicament according to any one of Claims 6 to 10, wherein the therapeutically effective amount of the carbamate compound of Formula 1 is 50 to 500 mg based on the free form once-daily administration.

12. A medicament for the alleviation or treatment of Angelman syndrome, comprising a therapeutically effective amount of a carbamate compound of the following Formula 1, or a pharmaceutically acceptable salt, solvate or hydrate thereof: 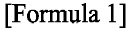 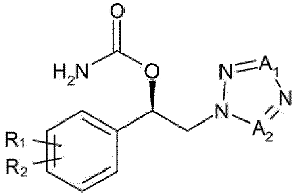 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

13. The medicament according to Claim 12, wherein the carbamate compound of Formula 1 is carbamic acid (R)-1-(2-chlorophenyl)-2-tetrazol-2-yl-ethyl ester of the following Formula 2:

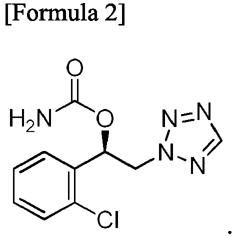

14. The medicament according to Claim 12, which is for use in the prevention, alleviation or treatment of symptoms of Angelman syndrome.

15. The medicament according to Claim 14, wherein the symptom of Angelman syndrome is developmental delay, learning disability, sociobehavioral disorder or seizure.

16. The medicament according to Claim 12, which is for use in the alleviation or treatment of autism spectrum disorder caused by Angelman syndrome, or autism spectrum disorder showing symptoms similar to Angelman syndrome.

17. The medicament according to any one of Claims 12 to 16, wherein the therapeutically effective amount of the carbamate compound of Formula 1 is 50 to 500 mg based on the free form once-daily administration.

18. A medicament for the alleviation or treatment of Rett syndrome, comprising a therapeutically effective amount of a carbamate compound of the following Formula 1, or a pharmaceutically acceptable salt, solvate or hydrate thereof: 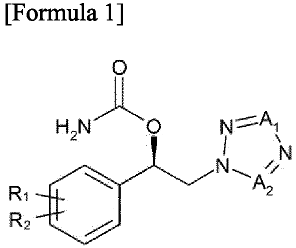 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

19. The medicament according to Claim 18, wherein the carbamate compound of Formula 1 is carbamic acid (R)-1-(2-chlorophenyl)-2-tetrazol-2-yl-ethyl ester of the following Formula 2:

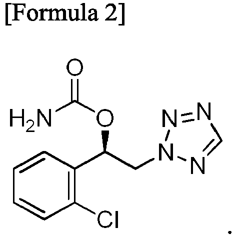

20. The medicament according to Claim 18, which is for use in the prevention, alleviation or treatment of symptoms of Rett syndrome.

21. The medicament according to Claim 20, wherein the symptom of Rett syndrome is developmental delay, learning disability, sociobehavioral disorder or seizure.

22. The medicament according to Claim 18, which is for use in the alleviation or treatment of autism spectrum disorder caused by Rett syndrome, or autism spectrum disorder showing symptoms similar to Rett syndrome.

23. The medicament according to any one of Claims 18 to 22, wherein the therapeutically effective amount of the carbamate compound of Formula 1 is 50 to 500 mg based on the free form once-daily administration.
